# FASCICULE DE BREVET EUROPEEN

(11) **EP 3 631 676 B1**
(45) Date de publication et mention de la délivrance du brevet: **30.07.2025**
(21) Numéro de dépôt: 18724281.3
(22) Date de dépôt: 22.05.2018
(51) Int. Cl.: G06F 18/00

(54) **PROCEDE ET UN SYSTEME DE STIMULATION D'UN INDIVIDU POUR FAVORISER UNE UTILISATION D'UN MEMBRE PARETIQUE PAR RAPPORT A UN MEMBRE SAIN**
VERFAHREN UND SYSTEM ZUR STIMULIERUNG EINES INDIVIDUUMS ZUR FÖRDERUNG DER NUTZUNG EINES PARETISCHEN MITGLIEDS IN BEZUG AUF EIN GESUNDES MITGLIED
METHOD AND SYSTEM FOR STIMULATING AN INDIVIDUAL TO PROMOTE USE OF A PARETIC MEMBER IN RELATION TO A HEALTHY MEMBER

(30) Priorité: 24.05.2017 FR 1754623
(43) Date de publication de la demande: 08.04.2020
(73) Titulaire: Universite D'Angers, 49035 Angers Cedex (FR); Centre Hospitalier Universitaire D'angers, 49933 Angers cedex 09 (FR)
(72) Inventeur: DINOMAIS, Micka l, 49460 Feneu (FR)
(74) Mandataire: Cabinet Le Guen Maillet
(86) Numéro de dépôt international: PCT/EP2018/063350
(87) Numéro de publication internationale: WO 2018/215443

(56) Documents cités:
- WO-A1-2007/142588
- US-A1- 2016 131 677
- US-B2- 8 706 241
- DANA R ANABY ET AL: "Assistive Technology for physical disability: Switches", 1 April 2016 (2016-04-01), XP055458748, Retrieved from the Internet <URL:https://www.medicalhomeportal.org/living-with-child/assistive-technology/switches> [retrieved on 20180313]
- "Toys and Technology for rehabilitation in cerebral palsy patients", 1 July 2008 (2008-07-01), XP002779029, Retrieved from the Internet <URL:https://www.eurekalert.org/pub_releases/2008-07/l-tat070108.php> [retrieved on 20180313]

## Description

La présente invention concerne un procédé et un système de stimulation d'un individu pour favoriser une utilisation d'un membre parétique par rapport à un membre sain.

La prise en charge des troubles moteurs dans le cas de la paralysie cérébrale est actuellement un challenge rééducatif.

La plasticité cérébrale est un processus continu permettant des modifications à moyen et long termes de l'organisation synaptique pour une meilleure efficacité des réseaux neuronaux. La neuro-plasticité est un mécanisme qui permet au cerveau d'adapter son organisation à différents niveaux en fonction des évènements extérieurs et ce afin d'être le plus efficient possible. La neuro-plasticité sous-tend des processus d'apprentissage et de mémorisation mais aussi des processus de réorganisation cérébrale suite à une lésion de l'encéphale.

En cas de paralysie cérébrale unilatérale, la mise en place d'une immobilisation d'un membre sain, et donc une surutilisation du membre parétique par l'individu, peut augmenter le volume de substance grise au niveau du cortex sensorimoteur de l'hémisphère lésé.

La paralysie cérébrale est actuellement la cause la plus commune de déficience motrice chez les enfants. La paralysie cérébrale est un trouble moteur non évolutif suite à une lésion cérébrale survenue sur le cerveau en développement. Ces lésions cérébrales précoces, intervenant dans la vie fœtale ou pendant la période néonatale peuvent altérer la mise en place et le fonctionnement du système sensorimoteur.

Le diagnostic de paralysie cérébrale est fait à un âge variable selon la gravité de l'atteinte. Un retard dans le développement moteur peut être observé par les parents dès les premiers mois de vie : absence de tenue de tête à 3 mois, de tenue assise à 9 mois, utilisation préférentielle d'une seule main.

Il est souhaitable d'effectuer une prise en charge rééducative motrice la plus précoce possible.

La prise en charge rééducative précoce a pour objectif de prévenir l'apparition secondaire de complications liées à la présence des déficits, d'améliorer la ou les fonctions déficitaires, de favoriser au mieux les activités de l'enfant et de permettre ainsi une participation sociale de l'enfant aussi bonne que possible.

La rééducation précoce est bien souvent effectuée en bloquant le membre sain dans le cadre de consultations et/ou prise en charge rééducative dans un milieu médicalisé spécialisé. Ces interventions peuvent générer de la frustration chez l'individu.

Le document Dana R Anaby et AL. intitulé « Assistive technology for physical disability : switches » décrit des jouets comprenant des interrupteurs et des détecteurs de proximité qui fonctionnent grâce à la force électromagnétique reçue de la proximité 10 d'une partie du corps d'un patient.

Le document US 8 706 241 B2 décrit un système de stimulation neuronale pour un patient en interaction avec un objet du quotidien.

La présente invention a pour but de résoudre les inconvénients de l'art antérieur en proposant un procédé et un système de stimulation d'un individu pour favoriser une utilisation d'un membre parétique par rapport à un membre sain au quotidien sans avoir à bloquer le membre sain, sans assistance technique, et dans le milieu de l'individu de manière à favoriser l'acceptation, l'observance et la compliance de l'individu.

A cette fin, selon un premier aspect, l'invention propose un système de stimulation d'un individu pour favoriser une utilisation d'un membre parétique par rapport à un membre sain, caractérisé en ce que le système comporte :
- un élément placé sur le membre parétique de l'individu,
- un objet, placé à proximité de l'individu, comprenant :
- un capteur de proximité déterminant la distance séparant l'élément placé sur le membre parétique et l'objet,
- des moyens d'activation d'un scénario pour la génération d'au moins un signal sonore et/ou visuel et/ ou somesthésique par l'objet, si la distance séparant l'élément placé sur le membre parétique et l'objet est inférieure à une distance donnée,
- des moyens de mémorisation et de mise à jour d'informations à chaque fois que la distance entre l'élément placé sur le membre parétique et l'objet est inférieure à la distance donnée.

Ainsi, la présente invention favorise une utilisation d'un membre parétique par rapport à un membre sain au quotidien sans avoir à bloquer le membre sain, sans assistance technique, et dans le milieu de l'individu de manière à favoriser l'acceptation, l'observance et la compliance de l'individu.

En mémorisant les informations, le praticien lors de la consultation de ces informations, peut ainsi faire le lien entre d'éventuels progrès de l'individu et l'utilisation du membre parétique.

La présente invention, en activant un scénario pour la génération d'au moins un signal sonore et/ou visuel et/ ou somesthésique si la distance séparant l'élément placé sur le membre parétique et l'objet est inférieure à une distance donnée et non lorsque le membre sain est proche de l'objet, stimule l'utilisation du membre parétique en générant une récompense pour l'individu.

Selon un mode particulier de l'invention, les informations mises à jour sont le nombre de fois où la distance entre l'élément placé sur le membre parétique et l'objet est inférieure à la distance donnée, les informations mémorisées sont la distance donnée et/ou le scénario et/ou un horodatage.

Ainsi, le praticien lors de la consultation de ces informations, peut faire le lien entre d'éventuels progrès de l'individu et l'utilisation du membre parétique.

Selon un mode particulier de l'invention, l'objet comporte en outre des moyens de modification de la distance donnée si le nombre de fois où la distance entre l'élément placé sur le membre parétique et l'objet est inférieure à la distance donnée est supérieur à une valeur prédéterminée.

Ainsi, la présente invention favorise l'apprentissage moteur. La distance donnée est par exemple importante dans un premier temps pour inciter l'individu à pratiquer l'exercice, puis est réduite au fur et à mesure de l'apprentissage

Selon un mode particulier de l'invention, l'objet comporte en outre des moyens de modification du scénario si la distance donnée est modifiée.

Ainsi, la présente invention stimule l'individu en faisant évoluer les scénarios au cours du temps.

Selon un mode particulier de l'invention, l'objet comporte en outre des moyens d'activation du scénario si un déplacement de l'objet est détecté et des moyens de modification du scénario si un nombre prédéterminé de déplacements de l'objet est détecté.

Ainsi, la présente invention stimule l'individu en faisant évoluer les scénarios au cours du temps selon la complexité des conditions à réaliser pour activer ou non la récompense.

Selon un mode particulier de l'invention, l'individu a un âge inférieur à 3 ans.

Ainsi, la présente invention est adaptée à la rééducation précoce chez l'enfant.

Selon un mode particulier de l'invention, l'élément est compris dans une orthèse et l'objet est une peluche.

Ainsi, la présente invention est adaptée à la rééducation précoce chez l'enfant.

Selon un mode particulier de l'invention, l'élément est compris dans une orthèse et l'objet est une poupée.

Ainsi, la présente invention est adaptée à la rééducation de l'enfant.

Selon un mode particulier de l'invention, l'élément est compris dans une orthèse et l'objet est un modèle réduit de véhicule.

Ainsi, la présente invention est adaptée à la rééducation de l'enfant.

Selon un mode particulier de l'invention, l'élément est compris dans une orthèse et l'objet est une balle et/ou un ballon.

Ainsi, la présente invention est adaptée à la rééducation motrice de l'hémiplégie.

Selon un mode particulier de l'invention, l'élément est un aimant permanent.

Ainsi, le coût de fabrication du système est réduit.

Selon un mode particulier de l'invention, l'objet comporte en outre des moyens de communication pour le transfert à un dispositif distant des informations mémorisées et mises à jour.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
la Fig. 1 représente un système de stimulation d'un individu pour favoriser une utilisation d'un membre parétique par rapport à un membre sain selon la présente invention ;
la Fig. 2 représente un exemple d'architecture électronique de l'objet selon la présente invention ;
la Fig. 3 représente un algorithme de stimulation d'un individu pour favoriser une utilisation d'un membre parétique par rapport à un membre sain selon la présente invention.

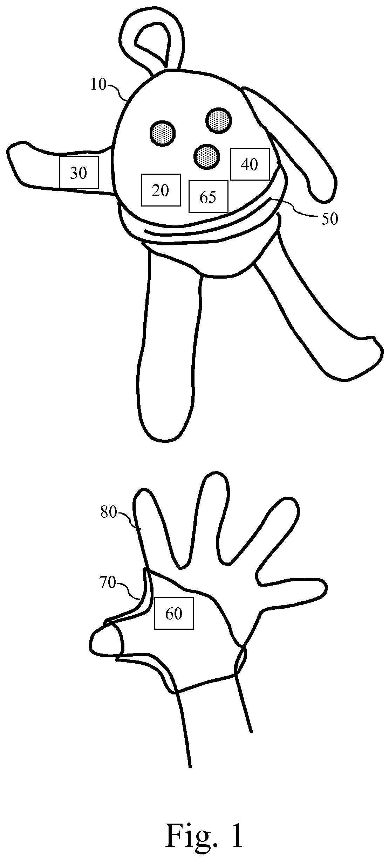

La **Fig. 1** représente un système un système de stimulation d'un individu pour favoriser une utilisation d'un membre parétique par rapport à un membre sain selon la présente invention.

Le système comporte un objet 10 et un élément 60 placé sur un membre parétique 80.

Dans l'exemple de la Fig. 1, le membre parétique est une main 80. Bien entendu la présente invention trouve son application avec d'autres membres.

Dans l'exemple de la Fig. 1, l'élément 60 est placé dans une orthèse 70. L'orthèse est par exemple utilisée lorsque le membre parétique a un pouce adductus. En effet, la plupart des enfants ayant une paralysie cérébrale présentent un pouce adductus, apparaissant très tôt du fait de la spasticité des muscles extrinsèques ou plus rarement des muscles intrinsèques de la main.

Bien entendu, l'élément 60 peut être placé sur un bracelet ou une bague.

L'élément 60 est par exemple un aimant permanent.

L'objet 10 est, dans l'exemple de la Fig. 1, une peluche. L'objet a une forme adaptée à l'âge de l'individu. Lorsque l'individu est un nourrisson, l'objet est une peluche. Bien entendu l'objet peut prendre de multiples formes comme par exemple, une poupée, un modèle réduit de véhicule, une balle ou un ballon.

L'objet 10 comporte un capteur de proximité 30 qui permet la détermination de la distance entre l'objet 10 et l'élément 60. Le capteur de proximité est par exemple un détecteur magnétique lorsque l'élément est un aimant.

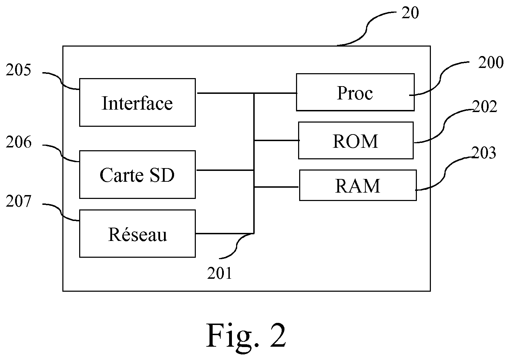

L'objet 10 comporte une carte électronique 20 qui sera décrite plus en détail en référence à la Fig. 2.

L'objet 10 comporte un détecteur de déplacement 65 tel que par exemple un accéléromètre et/ou un gyroscope.

L'objet 10 comporte des moyens d'émission de signaux sonores 40 et des moyens d'émission de signaux lumineux 50 tels que par exemple une barre de diodes électroluminescentes.

L'objet comporte aussi des moyens, non représentés en Fig. 1, de génération d'un signal somesthésique comme la génération de vibrations ou la génération de chaleur modérée.

La **Fig. 2** représente un exemple d'architecture électronique de l'objet selon la présente invention.

L'objet 10, plus précisément la carte électronique 20 de l'objet, comprend :
- un processeur, micro-processeur, ou microcontrôleur 200 ;
- une mémoire volatile 203 ;
- une mémoire non volatile 202 ;
- une interface entrée-sortie 205 ;
- un support de stockage amovible 206 tel que par exemple une carte SD ;
- une interface réseau 207 de type filaire ou bluetooth permet de communiquer avec un dispositif distant ;
- un bus de communication reliant le processeur 200 à la mémoire ROM 203, à la mémoire RAM 203, à l'interface entrée sortie 205, au support de stockage amovible 206 et à l'interface réseau 207.

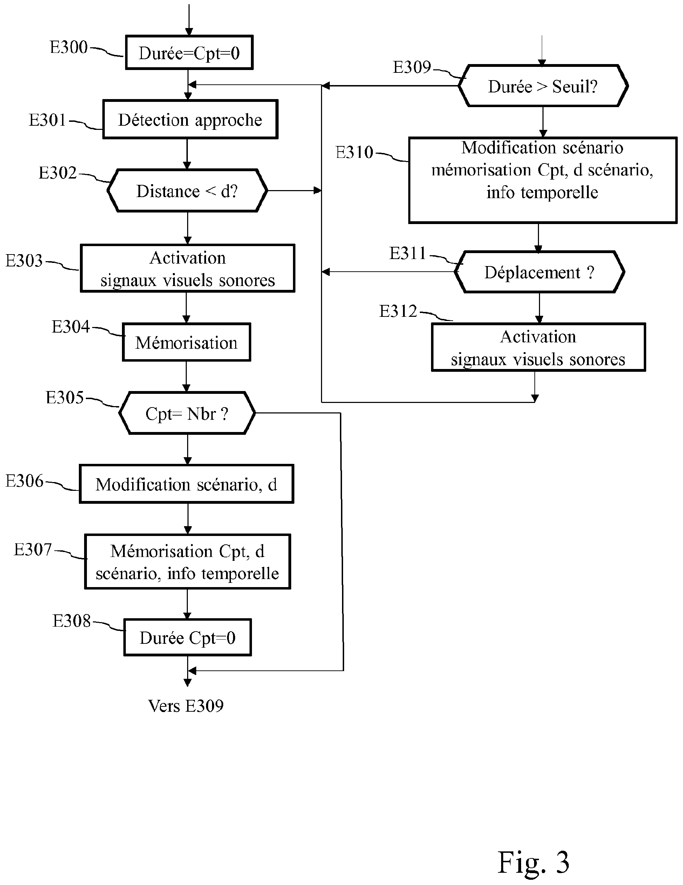

Le processeur 200 est capable d'exécuter des instructions chargées dans la mémoire volatile 203 à partir de la mémoire non volatile 202, d'une mémoire externe (non représentée), d'un support de stockage, tel qu'une carte SD ou autre, ou d'un réseau de communication. Lorsque l'objet 10 est mis sous tension, le processeur 200 est capable de lire de la mémoire volatile 203 des instructions et de les exécuter. Ces instructions forment un programme d'ordinateur qui cause la mise en œuvre, par le processeur 200, de tout ou partie du procédé décrit en relation avec la Fig. 3.

Tout ou partie du procédé décrit en relation avec la Fig. 3 peut être implémenté sous forme logicielle par exécution d'un ensemble d'instructions par une machine programmable telle qu'un DSP (*Digital Signal Processor* en anglais ou *Unité de Traitement de Signal Numérique* en français) ou un microcontrôleur, ou être implémenté sous forme matérielle par une machine ou un composant dédié, tel qu'un FPGA (*Field-Programmable Gate Array* en anglais ou *Matrice de Portes Programmable sur le Terrain* en français) ou un ASIC (*Application-Specific Integrated Circuit* en anglais ou *Circuit Intégré Spécifique à une Application* en français).

La **Fig. 3** représente un algorithme de de stimulation d'un individu pour favoriser une utilisation d'un membre parétique par rapport à un membre sain selon la présente invention.

Le présent algorithme est décrit dans un exemple dans lequel il est exécuté par le processeur 200.

A l'étape E300, le processeur 200 initialise une variable notée Durée et une variable Cpt à la valeur nulle. La variable durée est une variable représentative de la valeur d'une horloge qui est utilisée pour changer de scénario périodiquement. La variable Cpt est un compteur qui comptabilise le nombre de fois où la distance séparant l'élément placé sur le membre parétique et l'objet est inférieure à une distance donnée.

A l'étape E301, le processeur 200 obtient, par l'intermédiaire de l'interface entrée sortie 205, le signal de sortie du capteur de proximité 30.

A l'étape E302, le processeur 200 détermine, à partir du signal de sortie du capteur de proximité 30, la distance séparant l'objet 10, plus précisément le capteur de proximité 30, de l'élément 60. Le processeur 200 vérifie si la distance séparant l'élément placé sur le membre parétique et l'objet est inférieure à la distance donnée, par exemple de 20 cm.

Si la distance séparant l'élément placé sur le membre parétique 80 et l'objet 10 est inférieure à la distance donnée, le processeur 200 passe à l'étape E303.

Dans la négative, le processeur 200 retourne à l'étape E301.

A l'étape E303, le processeur 200 déclenche un scénario pour la génération d'au moins un signal sonore et/ou visuel et/ou somesthésique.

A l'étape E304, le processeur 200 mémorise et met à jour des informations.

Les informations mises à jour sont le nombre de fois Cpt où la distance entre l'élément placé sur le membre parétique et l'objet est inférieure à la distance donnée, les informations mémorisées sont la distance donnée et/ou le scénario et/ou un horodatage.

A l'étape E305, le processeur 200 vérifie si la variable Cpt est égale à une valeur prédéterminée Nbr.

Par exemple, Nbr est égale à 20.

Si la variable Cpt est égale à une valeur prédéterminée Nbr, le processeur 200 passe à l'étape E306. Dans la négative, le processeur 200 passe à l'étape E309.

A l'étape E306, le processeur 200 modifie le scénario.

Un scénario comporte une séquence de sons et une séquence d'animations visuelles telles qu'un clignotement des diodes électroluminescentes à un rythme variable, des variations dans les couleurs de la barre de diodes électroluminescentes.

A l'étape E307, le processeur 200 mémorise des informations.

Les informations mémorisées sont la distance donnée et/ou le scénario et/ou un horodatage.

A l'étape E308, le processeur 200 met la variable Cpt à la valeur nulle.

A l'étape E309, le processeur 200 vérifie si la variable Durée est supérieure à un seuil par exemple égal à 24 heures.

Si la variable Durée est supérieure au seuil, le processeur 200 passe à l'étape E310 et met la variable Durée à la valeur nulle. Dans la négative, le processeur 200 retourne à l'étape E301.

A l'étape E310, le processeur 200 modifie le scénario, mémorise et met à jour des informations.

Les informations mises à jour sont le nombre de fois Cpt où la distance entre l'élément placé sur le membre parétique et l'objet est inférieure à la distance donnée, les informations mémorisées sont la distance donnée et/ou le scénario et/ou un horodatage.

A l'étape E311, le processeur 200 vérifie si un déplacement de l'objet est détecté par le détecteur de déplacement 60.

Si un déplacement de l'objet est détecté, le processeur 200 passe à l'étape E312. Dans la négative, le processeur 200 retourne à l'étape E301.

A l'étape E312, le processeur 200 déclenche un scénario pour la génération d'au moins un signal sonore et/ou visuel et/ou somesthésique.

Le processeur 200 retourne ensuite à l'étape E301.

Bien entendu, la présente invention n'est nullement limitée aux modes de réalisation décrits ici, mais englobe, bien au contraire, toute variante à la portée de l'homme du métier.

En particulier, la présente invention dans un exemple dans lequel le processeur 200 modifie le scénario, mémorise et met à jour des informations en fonction du nombre de fois où la distance séparant l'élément placé sur le membre parétique et l'objet est inférieure à une distance donnée.

La modification du scénario, la mémorisation et la mise à jour des informations peut aussi être effectuée en fonction du nombre de déplacements de l'objet détecté.

## Revendications

1. Système de stimulation d'un individu pour favoriser une utilisation d'un membre parétique par rapport à un membre sain, **caractérisé en ce que** le système comporte :
- un élément (60) placé sur le membre parétique de l'individu,
- un objet (10), placé à proximité de l'individu, l'objet comprenant :
- un capteur de proximité (30) déterminant la distance séparant l'élément placé sur le membre parétique et l'objet,
- des moyens d'activation d'un scénario (20, 40, 50) pour la génération, par l'objet, d'au moins un signal sonore et/ou visuel et/ou somesthésique si la distance séparant l'élément placé sur le membre parétique et l'objet est inférieure à une distance donnée,
- des moyens de mémorisation (20) et de mise à jour d'informations à chaque fois que la distance entre l'élément placé sur le membre parétique et l'objet est inférieure à la distance donnée.

2. Système selon la revendication 1, **caractérisé en ce que** les informations mises à jour sont le nombre de fois où la distance entre l'élément placé sur le membre parétique et l'objet est inférieure à la distance donnée, les informations mémorisées sont la distance donnée et/ou le scénario et/ou un horodatage.

3. Système selon la revendication 2, **caractérisé en ce que** l'objet comporte en outre des moyens de modification de la distance donnée si le nombre de fois où la distance entre l'élément placé sur le membre parétique et l'objet est inférieure à la distance donnée est supérieur à une valeur prédéterminée.

4. Système selon la revendication 2, **caractérisé en ce que** l'objet comporte en outre des moyens de modification du scénario si la distance donnée est modifiée.

5. Système selon la revendication 2, **caractérisé en ce que** l'objet comporte en outre des moyens d'activation du scénario si un déplacement de l'objet est détecté et des moyens de modification du scénario si un nombre prédéterminé de déplacements de l'objet est détecté.

6. Système selon la revendication 5, **caractérisé en ce que** l'élément est compris dans une orthèse et l'objet est une peluche ou une poupée ou une balle ou un ballon ou un modèle réduit de véhicule.

7. Système selon la revendication 6, **caractérisé en ce que** l'élément est un aimant permanent.

8. Système selon l'une quelconque des revendications 1 à 7, **caractérisé en ce que** l'objet comporte en outre des moyens de communication pour le transfert à un dispositif distant des informations mémorisées et mises à jour.

## Patentansprüche

1. System zur Stimulation einer Person zum Begünstigen einer Benutzung eines paretischen Glieds im Verhältnis zu einem gesunden Glied, **dadurch gekennzeichnet, dass** das System Folgendes aufweist
- ein Element (60), das auf dem paretischen Glied der Person platziert ist,
- ein Objekt (10), das in der Nähe der Person platziert ist, wobei das Objekt Folgendes umfasst
- einen Näherungssensor (30), der den Abstand bestimmt, der das auf dem paretischen Glied platzierte Element und das Objekt trennt,
- Mittel zur Aktivierung eines Szenarios (20, 40, 50) für die Erzeugung wenigstens eines akustischen und/oder visuellen und/oder somatosensorischen Signals durch das Objekt, wenn der Abstand, der das auf dem paretischen Glied platzierte Element und das Objekt trennt, kleiner als ein bestimmter Abstand ist,
- Mittel zur Speicherung (20) und Aktualisierung von Informationen immer wenn der Abstand zwischen dem auf dem paretischen Glied platzierten Element und dem Objekt kleiner als der bestimmte Abstand ist.

2. System nach Anspruch 1, **dadurch gekennzeichnet, dass** die aktualisierten Informationen die Anzahl von Malen sind, die der Abstand zwischen dem auf dem paretischen Glied platzierten Element und dem Objekt kleiner als der bestimmte Abstand ist, die gespeicherten Informationen der bestimmte Abstand und/oder das Szenario und/oder ein Zeitstempel sind.

3. System nach Anspruch 2, **dadurch gekennzeichnet, dass** das Objekt ferner Mittel zum Ändern des bestimmten Abstands, wenn die Anzahl von Malen, die der Abstand zwischen dem auf dem paretischen Glied platzierten Element und dem Objekt kleiner als der bestimmte Abstand ist, größer als ein vorbestimmter Wert ist, aufweist.

4. System nach Anspruch 2, **dadurch gekennzeichnet, dass** das Objekt ferner Mittel zum Ändern des Szenarios, wenn der bestimmte Abstand geändert ist, aufweist.

5. System nach Anspruch 2, **dadurch gekennzeichnet, dass** das Objekt ferner Mittel zur Aktivierung des Szenarios bei Detektion einer Bewegung des Objekts und Mittel zum Ändern des Szenarios bei Detektion einer vorbestimmten Anzahl von Bewegungen des Objekts aufweist.

6. System nach Anspruch 5, **dadurch gekennzeichnet, dass** das Element in einer Orthese enthalten ist und das Objekt ein Plüschtier oder eine Puppe oder ein Ball oder ein Ballon oder ein Fahrzeugmodell ist.

7. System nach Anspruch 6, **dadurch gekennzeichnet, dass** das Element ein Dauermagnet ist.

8. System nach einem der Ansprüche 1 bis 7, **dadurch gekennzeichnet, dass** das Objekt ferner Kommunikationsmittel zur Übertragung der gespeicherten und aktualisierten Informationen an eine entfernte Vorrichtung aufweist.

## Claims

1. System for stimulating an individual in order to promote use of a paretic member with respect to a healthy member, **characterised in that** the system comprises:
- an element (60) placed on the paretic member of the individual,
- an object (10), placed in the vicinity of the individual, the object comprising:
- a proximity sensor (30) determining the distance separating the element placed on the paretic member and the object,
- means (20, 40, 50) for activating a scenario for generating, by the object, at least one audible and/or visual and/or somaesthetic signal if the distance separating the element placed on the paretic member and the object is less than a given distance,
- means (20) for storing and updating information whenever the distance between the element placed on the paretic member and the object is less than the given distance.

2. System according to claim 1, **characterised in that** the updated information is the number of times when the distance between the element placed on the paretic member and the object is less than the given distance, and the information stored is the given distance and/or the scenario and/or a timestamp.

3. System according to claim 2, **characterised in that** the object further comprises means for modifying the given distance if the number of times when the distance between the element placed on the paretic member and the object is less than the given distance is higher than a predetermined quantity.

4. System according to claim 2, **characterised in that** the object further comprises means for modifying the scenario if the given distance is modified.

5. System according to claim 2, **characterised in that** the object further comprises means for activating the scenario if a movement of the object is detected and means for modifying the scenario if a predetermined number of movements of the object are detected.

6. System according to claim 5, **characterised in that** the element is included in an orthosis and the object is a soft toy or a doll or a ball or a small-scale vehicle model.

7. System according to claim 6, **characterised in that** the element is a permanent magnet.

8. System according to any one of claims 1 to 7, **characterised in that** the object further comprises communication means for transferring stored and updated information to a remote device.
